# EUROPÄISCHE PATENTANMELDUNG

(11) **EP 2 374 411 A1**
(43) Veröffentlichungstag der Anmeldung: **12.10.2011**
(21) Anmeldenummer: 11161188.5
(22) Anmeldetag: 05.04.2011
(51) Int. Cl.: A61B 5/12

(54) **Vorrichtung zum beschleunigten Testen des Hörvermögens eines Patienten**

(30) Priorität: 06.04.2010 DE 202010004633 U
(71) Anmelder: Immenkemper, Martin, 59597 Erwitte (DE)
(72) Erfinder: Immenkemper, Martin, 59597 Erwitte (DE)
(74) Vertreter: Siekmann, Gunnar

(57) **Zusammenfassung**

Bei einer Vorrichtung zum beschleunigten Testen des Hörvermögens eines Patienten, umfassend zumindest eine Signalerzeugungseinrichtung sowie zumindest ein Schallaustrittselement, ist vorgesehen, dass die Signalerzeugungseinrichtung zumindest einen Speicherchip für reale Audio-Signale aufweist.

## Beschreibung

Die Erfindung betrifft eine Vorrichtung zum beschleunigten Testen des Hörvermögens eines Patienten, umfassend zumindest eine Signalerzeugungseinrichtung sowie zumindest ein Schallaustrittselement.

Patienten mit Hörproblemen können sich an Ohrenärzte sowie Hörgeräteakustiker wenden, um ihr Hörvermögen zu testen. Dies kann in aufwändigen Laboruntersuchungen erfolgen. Neben diesen aufwändigen Laboruntersuchungen ist es jedoch häufig wünschenswert, einen schnellen Test des Hörvermögens vornehmen zu können, um zunächst einmal überhaupt festzustellen, ob eine Person ein reduziertes Hörvermögen hat oder nicht.

Beschleunigte Teste bzw. Schnellteste werden daher mit kompakten Apparaturen angeboten. Im Stand der Technik werden Signalgeneratoren vorgesehen, welche Piep- und andere künstliche Geräusche erzeugen, die dann dem Untersuchten zugeführt werden.

Es hat sich gezeigt, dass derartige Geräte mit Signalgeneratoren bei den Patienten nicht besonders beliebt sind. Die Geräusche werden als synthetisch empfunden, sie haben mit dem realen Leben wenig gemein.

Der Erfindung liegt daher die Aufgabe zu Grunde, eine Vorrichtung der eingangs genannten Gattung aufzuzeigen, die von den Patienten besser akzeptiert wird.

Diese Aufgabe ist erfindungsgemäß dadurch gelöst, dass die Signalerzeugungseinrichtung zumindest einen Speicherchip für reale Audio-Signale aufweist.

Bei der erfindungsgemäßen Vorrichtung wird der Signalgenerator durch wenigstens einen Speicherchip ersetzt. Auf dem Speicherchip können reale Audio-Signale gespeichert werden, die dann in einem beschleunigten Test dem Patienten über das Schallaustrittselement zugeführt werden. Reale Audio-Signale können Gespräche sein, Verkehrssituationen entsprechen, Stadionatmosphären wiedergeben und ähnliches. Mithin werden dem Patienten Töne aus dem realen Leben zugeführt, in diesem soll ja das Gehör des Patienten ausreichend funktionieren. Herkömmliche Signalformen (natürliche und synthetische) können technisch eingebunden werden. Für einige Anwendungszwecke können mit dieser Vorrichtung auch synthetische Signalformen zur Verfügung gestellt werden. Ist der Patient bereits mit einem Hörgerät ausgestattet, kann er auf einfache und schnelle Weise überprüfen, ob die Einstellung dieses Hörgerätes richtig ist. Die Lautstärke der Wiedergabe kann an die Umgebung angepasst werden, unterschiedliche Sprachen können z. B. für Textbeiträge hinterlegt werden, so dass auch Ausländern dieser Test in einer gewohnten Landessprache ermöglicht ist.

Mit der erfindungsgemäßen Vorrichtung ist ein schneller Test dahingehend möglich, bei Personen ohne Hörgerät das Hörvermögen zu überprüfen. Die Testdauer kann sich beispielsweise auf zwei Minuten belaufen. Mit der Vorrichtung ist ein Screening möglich, so dass diese Vorrichtung quasi als halb automatisches Audiometer arbeitet.

Nach einer ersten Weiterbildung der Erfindung ist vorgesehen, dass in der Signalerzeugungseinrichtung Speicherbausteine für Sprachanweisungen für den Patienten angeordnet sind. Mit diesen Sprachanweisungen ist ein Patient durch einen Sprachtest führbar, so dass der Patient letztendlich in der Lage ist, den beschleunigten Test selbstständig durchzuführen.

Das Ergebnis des Tests kann beispielsweise an einem Monitor oder an einem Multimedia-Player angezeigt werden. Anhand der Anzeige können dann dem Patienten weitere Maßnahmen zur gegebenenfalls notwendigen Verbesserung seines Hörvermögens gegeben werden.

Es ist auch denkbar, dass das Ergebnis des Tests ausgedruckt wird und bei Durchführung des Tests kein Hörgeräteakustiker zugegen ist. Dann kann es auch möglich sein, die Signalerzeugungseinrichtung mit einem Münzautomaten auszurüsten, um auf diese Weise die Wertigkeit des Tests zu erhöhen.

Neben einem Ausdruck ist eine drahtgebundene oder drahtlose Übermittlung eines Testergebnisses möglich, dann kann die Signalerzeugungseinrichtung beispielsweise mit einem GSM- oder LAN-Modul verknüpft sein.

Als Schallaustrittselemente schließlich sind Kopfhörer oder frei in einem Raum aufgestellte Lautsprecher möglich.

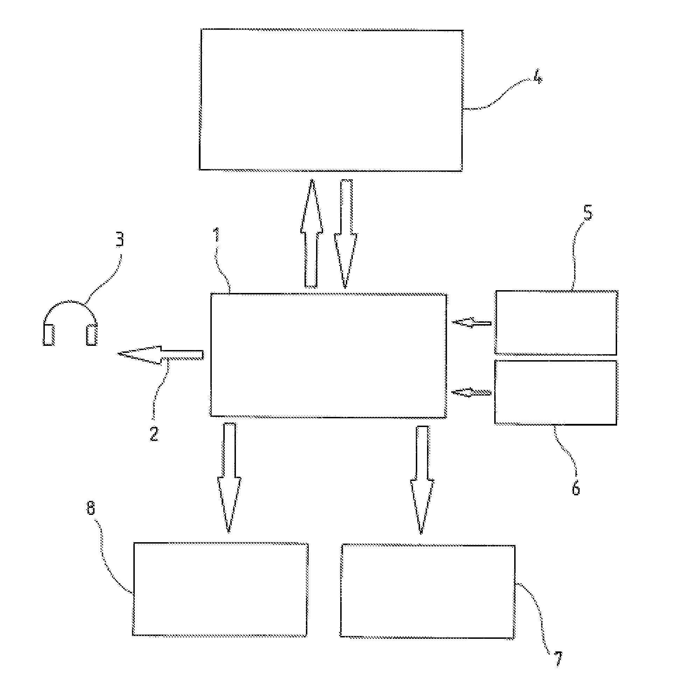

Ein Ausführungsbeispiel der Erfindung, aus dem sich weitere erfinderische Merkmale ergeben, ist in der Zeichnung dargestellt. Die einzige Figur der Zeichnung zeigt eine Vorrichtung zum beschleunigten Testen des Hörvermögens nach der Erfindung.

In der Zeichnung ist die erfindungsgemäße Vorrichtung schematisch dargestellt. Sie besteht aus einem Gehäuse 1, das über den Pfeil 2 mit einem Kopfhörer 3 verknüpft ist. Der Kopfhörer 3 dient als Schallaustrittselement für innerhalb des Gehäuses 1 erzeugte Signale. Im Gehäuse 1 ist die erfindungsgemäße Signalerzeugungseinrichtung angeordnet. Diese weist mindestens einen Speicherchip für reale Audio-Signale auf.

Die Signalerzeugungseinrichtung im Gehäuse 1 kann mit einem Monitor 4, mit einem Münzautomaten 5 und mit einer optionalen Tastatur 6 zum Eingeben von Kundendaten verknüpft sein.

Mit der erfindungsgemäßen Vorrichtung gewonnene Testergebnisse können entweder über einen Drucker 7 in Form von Hörkurven dem Patienten in die Hand gegeben werden, möglich ist auch die Versendung des Testergebnisses über Mobiltelefon oder über drahtlose Netze durch ein GSM- bzw. Wireless-Modul 8.

Die Verknüpfungen zwischen dem Gehäuse 1 und den einzelnen optionalen Bauelementen sind jeweils durch Pfeile bzw. Doppelpfeile verdeutlicht. Die Kommunikation zwischen der Signalerzeugungseinrichtung im Gehäuse 1 und dem Monitor ist dabei bidirektional ausgebildet, beispielsweise über eine RS-232 oder andere Schnittstellen, möglich sind auch drahtlose Netze.

## Patentansprüche

1. Vorrichtung zum beschleunigten Testen des Hörvermögens eines Patienten, umfassend zumindest eine Signalerzeugungseinrichtung sowie zumindest ein Schallaustrittselement,
**dadurch gekennzeichnet,**
**dass** die Signalerzeugungseinrichtung zumindest einen Speicherchip für reale Audio-Signale aufweist.

2. Vorrichtung nach Anspruch 1, **dadurch gekennzeichnet, dass** in der Signalerzeugungseinrichtung Speicherbausteine für Sprachanweisungen an den Patienten angeordnet sind.

3. Vorrichtung nach Anspruch 1 oder 2, **dadurch gekennzeichnet, dass** die Signalerzeugungseinrichtung mit einem Monitor (4) oder mit einem Multimediaplayer (4) verknüpfbar ist.

4. Vorrichtung nach einem der vorhergehenden Ansprüche, **dadurch gekennzeichnet, dass** die Signalerzeugungseinrichtung mit einem Münzautomaten (5) verknüpfbar ist.

5. Vorrichtung nach einem der vorhergehenden Ansprüche, **dadurch gekennzeichnet, dass** die Signalerzeugungseinrichtung mit einem Ticketdrucker (7) verknüpfbar ist.

6. Vorrichtung nach einem der vorhergehenden Ansprüche, **dadurch gekennzeichnet, dass** die Signalerzeugungseinrichtung mit einem GSM- oder LAN-Modul (8) verknüpfbar ist.

7. Vorrichtung nach einem der vorhergehenden Ansprüche, **dadurch gekennzeichnet, dass** das Schallaustrittselement als Kopfhörer (3) ausgebildet ist.

8. Vorrichtung nach einem der vorhergehenden Ansprüche, **dadurch gekennzeichnet, dass** das Schallaustrittselement als ein Lautsprecher oder als mehrere frei aufstellbare Lautsprecher ausgebildet ist.
